# EUROPÄISCHE PATENTANMELDUNG

(11) **EP 0 726 066 A2**
(43) Veröffentlichungstag der Anmeldung: **14.08.1996**
(21) Anmeldenummer: 96106328.6
(22) Anmeldetag: 19.01.1993
(51) Int. Cl.: A61F 2/34

(54) **Implantierbare Gelenkschale**

(30) Priorität: 31.01.1992 CH 287/92
(62) Teilanmeldung aus: 93810028.6
(71) Anmelder: SULZER Medizinaltechnik AG, CH-8404 Winterthur (CH)
(72) Erfinder: Koch, Rudolf, 8500 Frauenfeld (CH); Willi, Roland, 8413 Neftenbach (CH)
(74) Vertreter: Hammer, Bruno, Dr.

(57) **Zusammenfassung**

Mit der Erfindung ist eine verschleissfeste Innenschale (3) und die Entstehung ihrer Verbindung zu einer äusseren metallischen Schale (1) gezeigt. Die verschleissfeste Innenschale (3) wird auf ihrer Innenseite, die später eine Gelenkkugel aufnimmt, fertig bearbeitet und durch Schmelzfliessen über Vor- und Rücksprünge (4, 5, 6) lösbar mit einem Kunststoffrohling (2) verbunden. Dieser lässt sich problemlos auf eine endgültige Form mechanisch bearbeiten und mit einer äusseren metallischen Schale lösbar verbinden. Damit ist es auf einfache Art, nämlich durch spanende Formgebung, möglich unterschiedlich dimensionierte Zwischenschalen (2) zwischen verschleissfesten Innenschalen (3) und einer metallischen Aussenschale (1) vorzusehen.

## Beschreibung

Die Erfindung handelt von einer implantierbaren Gelenkschale, vorzugsweise einer Hüftgelenkschale, die eine äussere metallische Schale, welche zur Verankerung im Knochengewebe dient und eine verschleissfeste Innenschale zur Aufnahme eines Gelenkkopfes aufweist.

Die EP-A-0 453 694 zeigt eine dreiteilige künstliche Hüftgelenkschale, die eine äussere metallische Spreizschale aufweist, welche über ein Hilfswerkzeug in einer Knochenaushöhlung aufspreizbar ist, und die in der aufgespreizten Stellung durch eine Zwischenschale aus Polyethylen mit einem Bajonettverschluss gegen ein Zurückfedern der Verankerungselemente verriegelbar ist. In der Zwischenschale ist eine Innenschale, welche aus einem harten Werkstoff wie beispielsweise Aluminiumoxyd besteht, mit einer Schnappverriegelung verankert.
Schnappverriegelungen nutzen die Elastizität der beteiligten Partner aus, um über die Deformation von zueinander geführten Teilen eine definierte Rückhaltekraft zu erzeugen. Dies bedingt sehr genaue und in engen Toleranzen herzustellende Teile, um die gewünschte Verbindung zu erreichen, was für harte und somit schwer zu bearbeitende Innenschalen eine teure Herstellung verursacht.

Zweiteilige Gelenkschalen mit Schnappverriegelungen zwischen Aussen- und Innenschale sind seit vielen Jahren in Gebrauch. Sie erleichtern dem Operateur die Arbeit, indem die Aussenschale ohne Anwesenheit der Innenschale an einem Knochenbett befestigt werden kann und die Innenschale erst später einsetzbar ist. CH-PS 669 904 zeigt eine Anordnung mit einer Kunststoff-Innenschale, die in einer Aussenschale mit einer deformierbaren Schulter einschnappt. Wesentlich schwieriger wird in diesem Fall wie auch allgemein das Befestigungsproblem, wenn für die innerste Schale ein harter, schwer bearbeitbarer und unter Umständen auch spröder Werkstoff verwendet werden soll. Weder Elastizität noch Schlagfestigkeit bestimmter verschleissfester Materialien lassen eine sichere Schnappverbindung oder sichere andere Verbindungen zur Aussenschale zu.

Hier schafft die Erfindung Abhilfe. Sie löst die Aufgabe, Innenschalen aus schwer bearbeitbaren Werkstoffen mit einer Aussenschale zu verbinden. Diese Aufgabe wird mit den kennzeichnenden Merkmalen des unabhängigen Patentanspruchs 1 gelöst.

Die Vorteile der Erfindung sind darin zu sehen, dass der Bearbeitungsaufwand für die Innenschale wesentlich geringer wird, wenn bei gesinterten Werkstoffen, die im ungesinterten Zustand erreichbare Herstellgenauigkeit der Aussenkontur und wenn bei metallischen Werkstoffen, die im Rohling erreichbare Herstellgenauigkeit der Aussenkontur zum Aufbau einer Verbindung zur Aussenschale ausreichend sind. Ein weiterer Vorteil liegt in der einfachen Anpassbarkeit der Innenschale an verschiedene Ausführungsformen von metallischen Aussenschalen, selbst an implantierte Aussenschalen, bei denen die Innenschale ausgewechselt wird.

Bei grossen Stückzahlen lohnt es sich, passende Kunststoffrohlinge in einer Kunststoffspritzform zu erzeugen.

Ein weiterer Vorteil liegt in der Dämpfung des Kunststoffes, der Spannungsspitzen beim Uebertragen von Kräften abbaut. Weitere Vorteile ergeben sich aus den Kennzeichen der abhängigen Ansprüche 2 und 3.

Im folgenden wird die Erfindung anhand von Ausführungsbeispielen beschrieben. Es zeigen:
- Figur 1: Schematisch einen Schnitt in der Achse einer verschleissfesten Innenschale, die von einem angespritzten Kunststoffrohling umgeben ist;
- Figur 2: schematisch die Seitenansicht einer verschleissfesten Innenschale mit einer angespritzten und fertig bearbeiteten Kunststoffschale, die über eine Schnappverbindung mit einer äusseren metallischen Schale verbunden ist;
- Figur 3: schematisch die Seitenansicht einer vorgeheizten Innenschale vor dem Zusammenfügen mit einem zugehörigen Kunststoffrohling; und
- Figur 4: schematisch die Seitenansicht der zusammengefügten Teile aus Fig. 3 nach dem Fertigbearbeiten der Kunststoffschale.

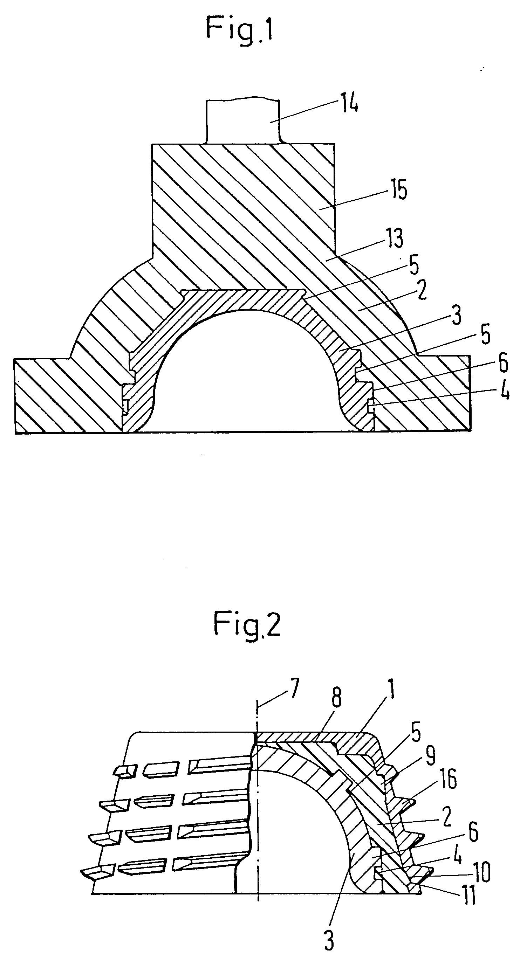

In den Figuren 1 und 2 ist eine verschleissfeste Innenschale 3 und die Entstehung ihrer Verbindung zu einer äusseren metallischen Schale 1 gezeigt. Die verschleissfeste Innenschale 3 wird auf ihrer Innenseite, die später eine Gelenkkugel aufnimmt, fertig bearbeitet, in eine passende Kunststoffspritzform eingelegt und mit einem angespritzten Kunststoffrohling versehen. Dieser ist durch ausgespritzte Vor- und Rücksprünge 4, 5, 6 in der verschleissfesten Innenschale 3 nicht mehr lösbar mit dieser verbunden. Die so geschaffene Verbindung ist für Kräfteübertragung geeignet. Eine so angespritzte Kunststoffschale 2 lässt sich problemlos auf ihre endgültige Form mechanisch bearbeiten und mit der äusseren metallischen Schale lösbar verbinden. Der Kunststoff ist einfach bearbeitbar und hat Dämpfungseigenschaften.

In Figur 1 ist ein Kunststoffrohling 13 auf einer auf der Innenseite fertig bearbeiteten, verschleissfesten Innenschale 3 aufgespritzt. Die Innenschale 3 besitzt auf ihrer umspritzten Aussenseite Vor- und Rücksprünge in Form von Rinnen 5, Bohrungen 4 und Vorsprüngen 6. Bei Innenschalen 3 aus metallischen Werkstoffen genügt es, diese Vor- und Rücksprünge in groben Toleranzen für das Umspritzen vorzubereiten. Bei Innenschalen 3 aus Sintergefüge, wie z.B. Metalloxiden genügt es von der Genauigkeit her die Vor- und Rücksprünge im ungesinterten Zustand zu erzeugen. Die nicht zu umspritzende Innenseite der Innenschale wird vor dem Umspritzen fertig bearbeitet. Anschliessend wird die Innenschale 3 in eine Kunststoffspritzform (nicht gezeigt) eingelegt und umspritzt. Figur 1 entspricht einem entformten Teil, das an seinem Anguss 14 abgetrennt wurde. Der Anguss 14 geht zunächst in ein Reservevolumen 15 über, aus dem flüssiges Material nachgesogen werden kann, wenn der Kunststoff auf der Innenschale 3 abkaltet. Der Kunststoffrohling 13 ist mit reichlicher Zugabe versehen, um durch mechanische Bearbeitung des Kunststoffs die Passungen zu verschiedenen Aussenschalen 1 in Form von unterschiedlich dimensionierten Kunststoffschalen 2 herzustellen.

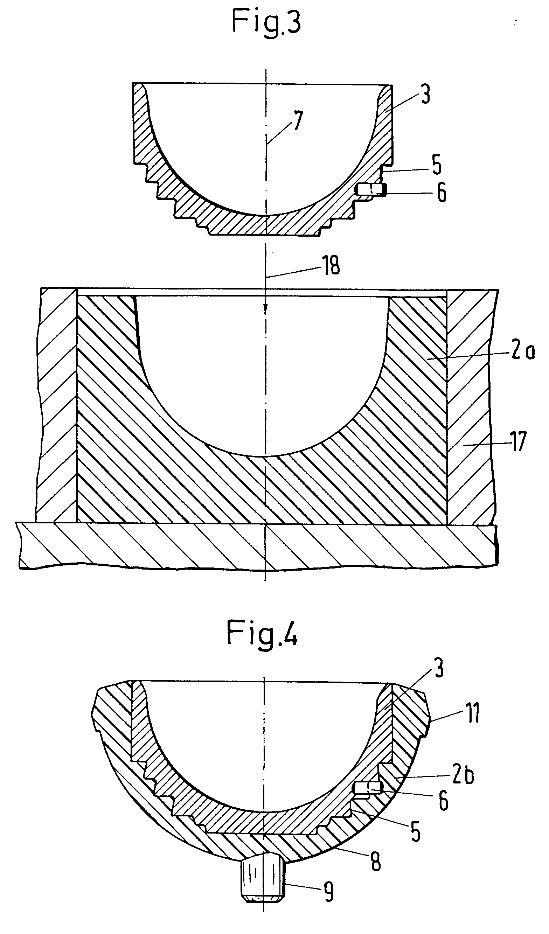

In Figur 3 ist eine verschleissfeste Innenschale 3 mit hinterschnittenen Rücksprüngen 5 auf der Aussenseite gezeigt. Ein eingepresster Stift steht als Vorsprung 6 vor, welcher später als Verdrehsicherung wie in Figur 4 wirkt. Ein Kunststoffrohling 2a mit zylindrischer Aussenform ist in einem Stützring 17 gefangen und besitzt eine Innenwölbung, welche der Hüllkurve der Aussenfläche der Innenschale 3 entspricht. Die Innenschale 3 wird in einer Einpressrichtung 18 in den Kunststoffrohling 2a eingepresst. Während oder kurz vor dem Einpressen wird die Innenwölbung angeschmolzen, um flüssigen Kunststoff in die Hinterschneidungen 5 fliessen zu lassen. Das Anschmelzen der Innenwölbung kann durch vorheriges Aufheizen der Innenschale 3 geschehen oder/und durch kurzzeitiges Aufheizen der Innenwölbung mit z.B. einem Heissluftstrom aus einer Düse (nicht gezeigt). Nach dem Einpressen der Innenschale 3 und dem Abkühlen des Kunststoffs ist eine dauerhafte Verbindung entstanden, die bei einer Aufnahme an der Innenschale 3 eine spanende Formgebung am Kunststoffrohling 2a ermöglicht.

Figur 4 und 2 zeigen eine Innenschale 3 mit fertig bearbeiteter Kunststoffschale 2b. Schnappverbindungen 11, Aufnahmeflächen 8, 9, 10 und Anschläge können durch die spanende Formgebung jeweils einer metallischen äusseren Schale 1 angepasst werden.
Figur 2 zeigt weiterhin eine Aussenschale 1 mit selbstschneidendem Gewinde 16 und eine Innenschale 3 mit Kunststoffschale 2, deren Kontaktflächen rotationssymmetrisch zur Polachse 7 liegen und jeweils in einer Aufspannung durch Drehen erzeugt werden können.

Die Wahl der Werkstoffe für die Innenschale 3 ist damit viel weniger von den Bearbeitungskosten beeinflusst und es kommen dafür auch schwer bearbeitende Kobalt-Chrom-Molybdänlegierungen, Kobalt-Chrom-Kohlenstofflegierungen und Sintergefüge wie z.B. aus Metalloxiden in Frage. Für die Lagerhaltung ergeben sich weitere Vorteile, indem bei einem Zwischenlager von mit Kunststoffrohlingen 2a versehenen verschleissfesten Innenschalen 3 unterschiedliche Aussenformen wegen der einfachen Bearbeitbarkeit als Fertigteile schnell verfügbar sind. Die Verbindung zwischen der äusseren metallischen Schale 1 und der Innenschale 3 mit Kunststoffschale 2b kann je nach Anforderung vorgenommen werden. Eine werkstattmässige Montage ist ebenso möglich, wie das Einsetzen einer Innenschale 3 mit Kunststoffschale 2b in eine bereits implantierte äussere metallische Schale. Innenschalen 3 mit Kunststoffschalen 2b können auf diese Weise auch als Ersatz für weniger verschleissfeste Innenschalen in bereits eingewachsene Aussenschalen eingesetzt werden.

## Patentansprüche

1. Implantierbare Gelenkschale, vorzugsweise Hüftgelenkschale, die eine äussere metallische Schale (1) zur Verankerung im Knochengewebe und eine verschleissfeste Innenschale (3) zur Aufnahme eines Gelenkkopfes aufweist, dadurch gekennzeichnet, dass Innenschalen (3) durch Schmelzfliessen über Rücksprünge (4, 5) mit Kunststoffschalen (2) verbunden sind, welche unterschiedlich dimensioniert sind und welche durch spanende Formgebung an die Form der äusseren metallischen Schale (1) angepasst und mit dieser lösbar verbindbar sind.

2. Implantierbare Gelenkschalen nach Anspruch 1, dadurch gekennzeichnet, dass die Kunststoffschalen (2) an verschiedene Ausführungsformen von metallischen Aussenschalen (1) angepasst sind.

3. Implantierbare Gelenkschalen nach Anspruch 1 oder 2, dadurch gekennzeichnet, dass die Kunststoffschalen (2) Kontaktflächen zur metallischen Aussenschale (1) aufweisen, welche rotationssymmetrisch zur Polachse (7) der Innenschale (3) liegen.
